(19) Europäisches Patentamt
European Patent Office
Office européen des brevets

(11) Publication number: **0 250 264 A1**

(12) **EUROPEAN PATENT APPLICATION**

(21) Application number: **87305476.1**

(22) Date of filing: **19.06.87**

(51) Int. Cl.⁴: **C 07 C 87/29**, C 07 C 93/14, C 07 C 85/20, C 07 C 57/42, C 07 C 59/66, C 07 C 51/00, C 07 F 7/08, C 07 C 125/065, A 61 K 31/135

(30) Priority: **19.06.86 US 876043**

(43) Date of publication of application: **23.12.87 Bulletin 87/52**

(84) Designated Contracting States: **AT BE CH DE ES FR GB GR IT LI LU NL SE**

(71) Applicant: **SMITHKLINE BECKMAN CORPORATION, One Franklin Plaza P O Box 7929, Philadelphia Pennsylvania 19103 (US)**

(72) Inventor: **Chambers, Pamela Anne, 286 Iven Avenue, St. Davids Pennsylvania 19087 (US)**
Inventor: **Kruse, Lawrence Ivan, 33 Firs Walk, Tewin Hertfordshire AL6 0NY (GB)**

(74) Representative: **Giddings, Peter John, Dr. et al, Smith Kline & French Laboratories Ltd. Corporate Patents Mundells, Welwyn Garden City Hertfordshire AL7 1EY (GB)**

(54) **Irreversible dopamine-Beta-hydroxylase inhibitors.**

(57) Substituted β-ethenyl and β-ethynyl benzeneethanamine compounds of structrue (I)

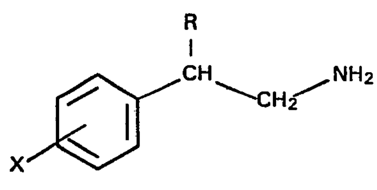

in which X is hydrogen or hydroxy; and R is ethenyl or ethynyl; are potent, irreversible inhibitors of mammalian dopamine-β-hydroxylase. Included are pharmaceutical compositions and methods for using these compounds to inhibit DH, and processes and intermediates used in preparing active compounds.

EP 0 250 264 A1

## TITLE

# IRREVERSIBLE DOPAMINE-ß-HYDROXYLASE INHIBITORS

## FIELD OF THE INVENTION

This invention relates to novel compounds that irreversibly inhibit dopamine-ß-hydroxylase, and novel pharmaceutical compositions, and methods for inhibiting dopamine-ß-hydroxylase.

## BACKGROUND OF THE INVENTION

In the catecholamine biosynthetic pathway, tyrosine is converted in three steps to norepinephrine (NE). Intermediates are dihydroxyphenylalanine (DOPA) and dopamine (DA). Dopamine is hydroxylated to norepinephrine by dopamine-ß-hydroxylase (DBH) in the presence of oxygen and ascorbic acid.

Inhibition of catecholamine activity decreases blood pressure. Weinshilboum, Mayo Clin. Proc. 55, 39 (1980), reviews compounds that inhibit catecholamine activity by acting upon adrenergic receptors. Alternatively, the catecholamine biosynthetic pathway can be suppressed at any of the three steps, resulting in reduced NE levels. In addition to producing an antihypertensive effect, inhibitors of NE synthesis are

active as diuretics, natriuretics, cardiotonics, and vasodilators. Inhibition of DBH activity can have the added advantage of increasing DA levels, which as reported by Ehrreich *et al.*, "New Antihypertensive Drugs," Spectrum Publishing, 1976, pp. 409-432, has selective vasodilator activity at certain concentrations.

DBH inhibitors also have been shown to reduce or prevent formation of gastric ulcers in rats by Hidaka *et al.*, "Catecholamine and Stress," edit. by Usdin *et al.*, Permagon Press, Oxford, 1976, pp. 159-165 and by Osumi *et al.*, *Japan J. Pharmacol.* *23*, 904 (1973).

A number of reversible DBH inhibitors are known. These generally are divided into two classes, namely, metal chelating agents, which bind copper in the enzyme, and phenethylamine analogues. Rosenberg *et al.*, "Essays in Neurochemistry and Neuropharmacology," Vol. 4, edit. by Youdim *et al.*, John Wiley & Sons, 1980, pp. 179-192, and Goldstein, *Pharmacol. Rev.* *18*(1), 77 (1966), review DBH inhibitors. The former report that many potent DBH inhibitors have a hydrophobic side chain of size comparable to the aromatic ring of DA, leading the authors to suggest that incorporation of a terminal hydroxyl group on a 4- to 6- carbon side chain on a phenethylamine analogue may yield potent inhibitors.

Known reversible DBH inhibitors include:

(a) 5-alkylpicolinic acids [See, Suda *et al.*, *Chem. Pharm. Bull* *17*, 2377 (1969); Umezawa *et al.*, *Biochem. Pharmacol.* *19*, 35 (1969); Hidaka *et al.*, *Mol. Pharmacol* *9*, 172 (1973); Miyano *et al.*, *Chem. Pharm. Bull.* *26*, 2328 (1978); Miyano *et al.*, *Heterocycles* *14*, 755 (1980); Claxton *et al.*, *Eur. J. Pharmacol.* *37*, 179 (1976)];

(b) BRL 8242 [See, Claxton *et al.*, *Eur. J. Pharmacol.* *37*, 179 (1976)];

(c) 1-alkylimidazole-2-thiols [See, Hanlon et al., Life Sci. 12, 417 (1973); Fuller et al., Adv. Enzyme Regul. 15, 267 (1976)];

(d) substituted thioureas [See, Johnson et al., J. Pharmacol. Exp. Ther. 168, 229 (1969)]; and

(e) benzyloxyamine and benzylhydrazine [See, Creveling et al., Biochim. Biophys. Acta 64, 125 (1962); Creveling et al., Biochim. Biophys. Acta 8, 215 (1962); Van Der Schoot et al., J. Pharmacol. Exp. Ther. 141, 74 (1963); Bloom, Ann. N.Y. Acad. Sci. 107, 878 (1963)].

Each of the above compounds except benzyloxyamine and benzylhydrazine apparently owes its inhibitory effect to metal chelating properties. Alkyl derivatives of imidazole-2-thiol are more potent, presumably due to non-specific interaction of the alkyl substituent with the enzyme. Benzyloxyamine and benzylhydrazine are phenethylamine analogues which apparently act as competitive inhibitors.

In addition to the above compounds, Runti et al., Il Farmaco Ed. Sci. 36, 260 (1980), report that other fusaric acid derivatives and analogues inhibit DBH. These include phenylpicolinic acid, which has twice the inhibitory activity of fusaric acid, and 5-(4-chlorobutyl) picolinic acid, and others such as substituted amides of fusaric acid and acids and amides of 5-butyroylpicolinic acid, 5-aminopicolinic acid and 5-hydrazinopicolinic acid, and derivatives thereof.

Hidaka et al., Molecular Pharmacology, 9, 172-177 (1972) report that 5-(3,4-dibromobutyl)picolinic acid and 5-(dimethyldithiocarbamoyl)methylpicolinic acid are DBH inhibitors.

Bupicomide, 5-(n-butyl)picolinamine, is reported by Ehrreich et al., "New Antihypertensive Drugs", Spectrum Publications, 1976, pg. 409-432, to be a DBH inhibitor that possesses the ability to lower blood pressure.

In European Patent Application No. 125,033 a series of 1-phenyl and 1-phenylalkylimidazole compounds having a mercapto or alkylthio group in the 2-position are disclosed. These compounds are described as having DBH inhibiting activity.

United States Patent No. 4,532,331 describes various 1-benzyl-2-aminomethylimidazole derivatives that inhibit DBH activity and includes pharmaceutical compositions containing these derivatives and methods of using these derivatives to inhibit DBH activity.

United States Patent No. 4,487,761 describes several methylpyridine derivatives isolated from the fermentation broth of a strain of Streptoverticillium. These compounds inhibit DBH activity.

Friedman et al., Psychosomatic Med. 40, 107 (1978), report that patients treated with alpha-methyl-DOPA, guanethidine, and reserpine, but not propranolol and diuretics, have lowered DBH levels, although the significance of the observation is uncertain.

Non-specific, often toxic effects of known DBH inhibitors have obviated clinical use of these compounds. Fusaric acid, for example, is hepatotoxic. See, for example, Teresawa et al., Japan. Cir. J. 35, 339 (1971) and references cited therein. Presumably, the picolinic acid structure interacts with a number of metalloproteins and enzymes non-specifically to produce the observed side effects.

## SUMMARY OF THE INVENTION

The present invention resides in the discovery that DBH is inhibited by substituted β-ethynyl and β-ethenyl benzeneethanamine compounds and that the DBH inhibition produced by these compounds is irreversible. These compounds are potent and produce prolonged DBH inhibition.

Presently preferred compounds of the invention include:

ß-ethynylbenzeneethanamine,
ß-ethynyl-3-hydroxybenzeneethanamine,
ß-ethynyl-4-hydroxybenzeneethanamine,
ß-ethenylbenzeneethanamine,
ß-ethenyl-3-hydroxybenzeneethanamine, and
ß-ethenyl-4-hydroxybenzeneethanamine.

## DETAILED DESCRIPTION OF THE INVENTION

The presently invented compounds that irreversibly inhibit DBH have the following formula:

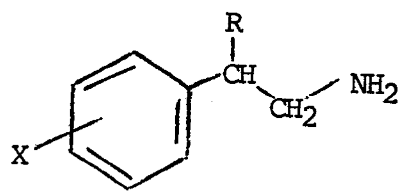

(I)

in which:

X is hydrogen or hydroxy; and
R is ethynyl or ethenyl;

or a pharmaceutically acceptable salt or hydrate thereof.

Compounds of Formula I are prepared from substituted phenylmethylene compounds by processes such as shown in Scheme I below. In Scheme I, $X^1$ is hydrogen or para-$C_{1-4}$ alkoxybenzyloxy, $R^2$ is 1,3-propanedioic acid di$C_{1-4}$ alkyl ester, preferably diethyl ester, or 2,2-dimethyl-1,3-dioxane-4,6-dione, and X is as described in Formula I, above.

Scheme I illustrates reaction of a substituted phenylmethylene compound (A) with a tri $C_{1-4}$ alkylsilylacetylene, Grignard, preferably trimethylsilylacetylene, followed by addition of a strong acid such as hydrochloric acid, to

prepare substituted phenyl(trimethylsilylethynyl)methyl-propanedioic acid diethyl esters (B) or substituted 2,2-dimethyl-5-phenyl(trimethylsilylethynylmethyl)-1,3-dioxane-4,6-diones (C).

Substituted ß-ethynylbenzenepropanoic acid compounds (D) then are prepared by heating at about 80 to 120°C formula (B) compounds with a strong base such as an alkali metal hydroxide, for example sodium hydroxide, acidifying this reaction mixture with strong acid, such as hydrochloric acid, to obtain the diacid, followed by heating at about 80 to 120°C the diacid with aqueous organic base such as triethylamine, aminopyridine, or, preferably, pyridine.

Formula (D) compounds also are prepared by heating at about 80 to 120°C a mixture of a formula (C) compound with an aqueous organic base such as triethylamine, aminopyridine, or, preferably, pyridine, then heating the mixture at about 40-60°C with potassium fluoride in a dipolar, aprotic solvent such as dimethylformamide, thereby producing a salt, and then acidifying the salt by addition of strong acid such as hydrochloric acid.

The substituted ß-ethynylbenzenepropanoic acid compounds (D) thus formed next are heated at about 80 to 120°C with diphenylphosphorylazide, an organic base, preferably triethylamine, and a 4-$C_{1-4}$alkoxybenzyl alcohol, such as 4-methoxybenzyl alcohol (PMBOH), to prepare substituted 2-(ethynyl-2-phenylethyl)carbamic acid, 4-methoxyphenylmethyl esters (E).

To prepare Formula I compounds having a ß-ethynyl group (F), formula (E) compounds are deprotected by addition of a saturated ethereal hydrochloride solution. Formula (I) compounds having a ß-ethenyl group (H) are formed by hydrogenation of formula (E) compounds over Lindlar catalyst (palladium on calcium carbonate poisoned

with lead) available from Aldrich Chemical Company to produce formula (G) compounds followed by deprotection of the formula (G) compounds by addition of a saturated ethereal hydrochloride solution.

Formula (I) compounds in which X is hydrogen or hydroxy at the 2 or 3 position can be prepared from formula (A) compounds wherein $R^2$ is a 1,3-propanedioic acid di$C_{1-4}$ alkyl ester or 2,2-dimethyl-1,3-dioxane-4,6-dione. According to the reactions of Scheme (I), however, Formula (I) compounds wherein X is 4-hydroxy must be formed from formula (A) compounds wherein $R^2$ is 2,2-dimethyl-1,3-dioxane-4,6-dione.

Starting formula (A) compounds that are phenylmethylene propanedioic acid di$C_{1-4}$alkyl esters (X is H) are available and can be prepared by known procedures. Formula (A) compounds having an para-$C_{1-4}$ alkoxybenzyloxy group are prepared from corresponding hydroxy compounds by alkylation with a suitable alkoxybenzyl halide, such as methoxybenzyl chloride, by known processes. The corresponding hydroxy compounds are available and can be prepared by known techniques.

Formula (A) compounds that are 2,2-dimethyl-1,3-dioxane-4,6-diones are prepared from corresponding alkoxybenzyloxybenzaldehydes by reaction with Meldrum's acid (2,2-dimethyl-1,3-dioxane-4,6-dione) according to known procedures. For example, a methoxybenzyloxybenzaldehyde in an organic solvent such as toluene is reacted with Meldrum's acid in the presence of glacial acetic acid and piperidine to yield methoxybenzyloxybenzaldehyde-1,3-dioxane-4,6-dione compounds of formula (A).

The present invention includes compounds of the following Formulae II, III, and IV that are useful in preparing Formula (I) compounds of the invention:

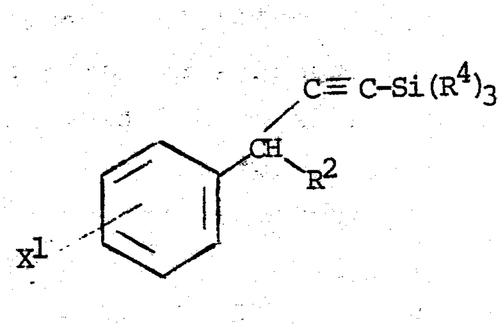

(II)

in which:

$X^1$ is H or 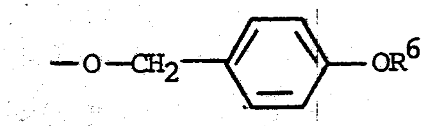

$R^6$ is $C_{1-4}$ alkyl;

$R^2$ is $CH(CO_2R^7)_2$ or 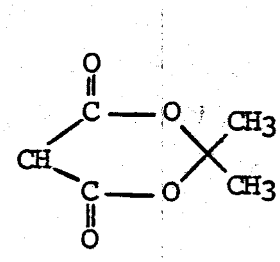 ;

$R^7$ is $C_{1-4}$alkyl; and

$R^4$ is $C_{1-4}$ alkyl;

provided that when $X^1$ is 4- 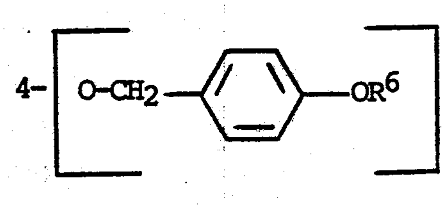 ,

$R^2$ is 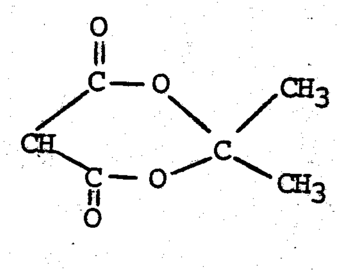 .

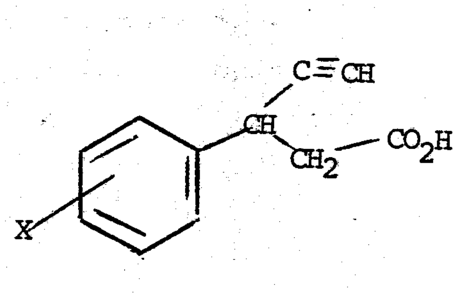

(III)

in which:

$X^1$ is H or 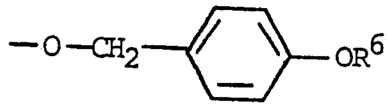 and $R^6$ is $C_{1-4}$ alkyl.

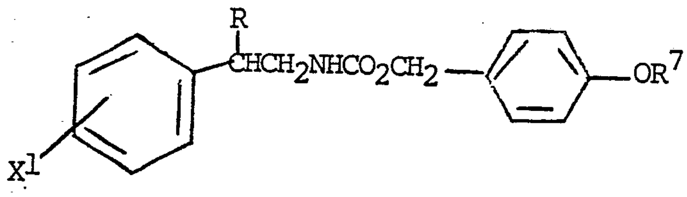

in which

$X^1$ is H or 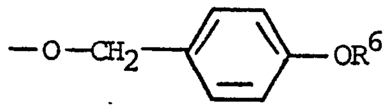 ;

$R^6$ and $R^7$ are $C_{1-4}$ alkyl; and

R is $-C{\equiv}CH$ or $-CH{=}CH_2$.

Also included in the invention are novel methods of synthesizing compounds of Formulae (II), (III), and (IV) as depicted in Scheme I above and described in the examples that follow.

Scheme I

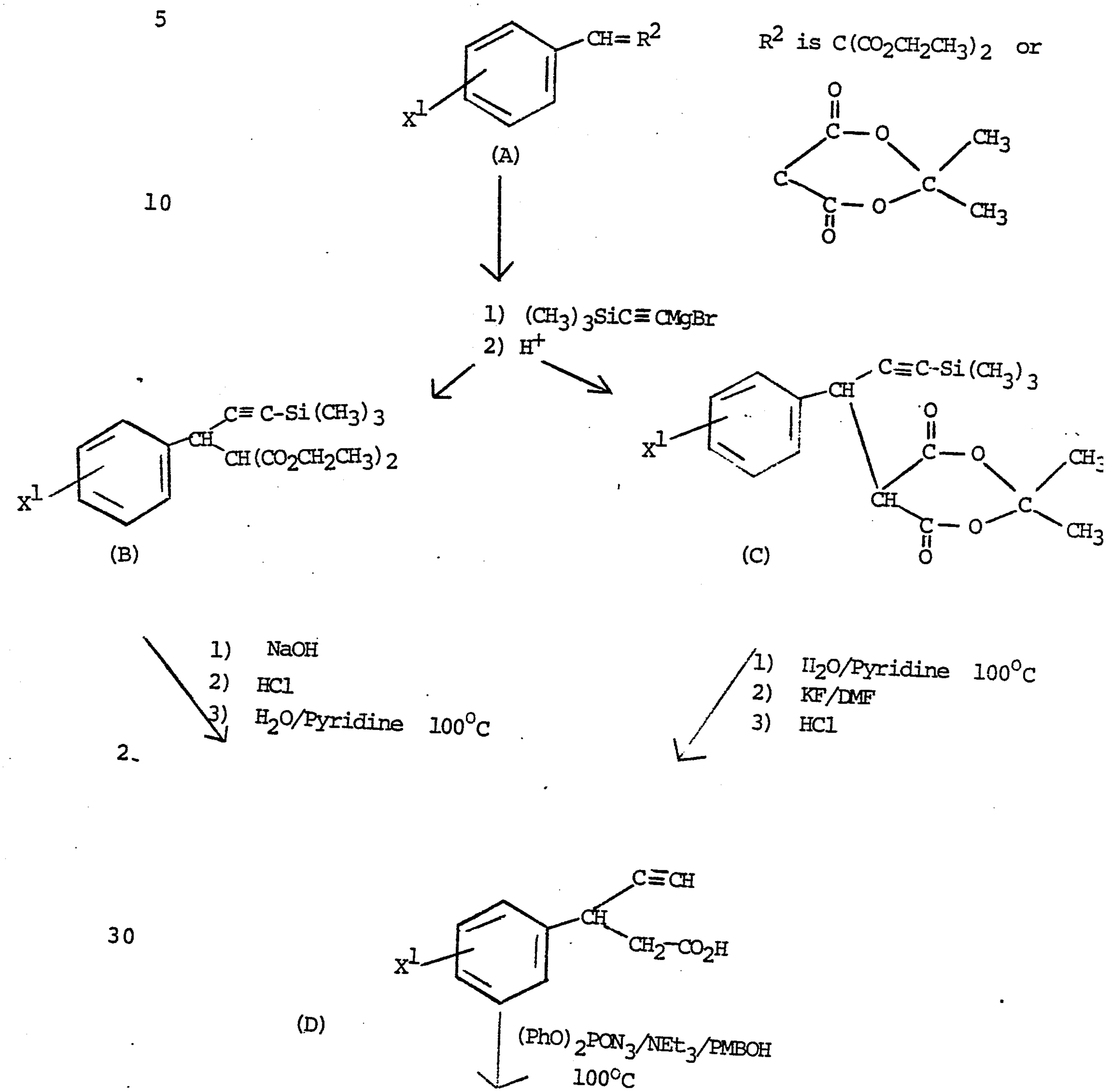


BAD ORIGINAL

Scheme I (Continued)

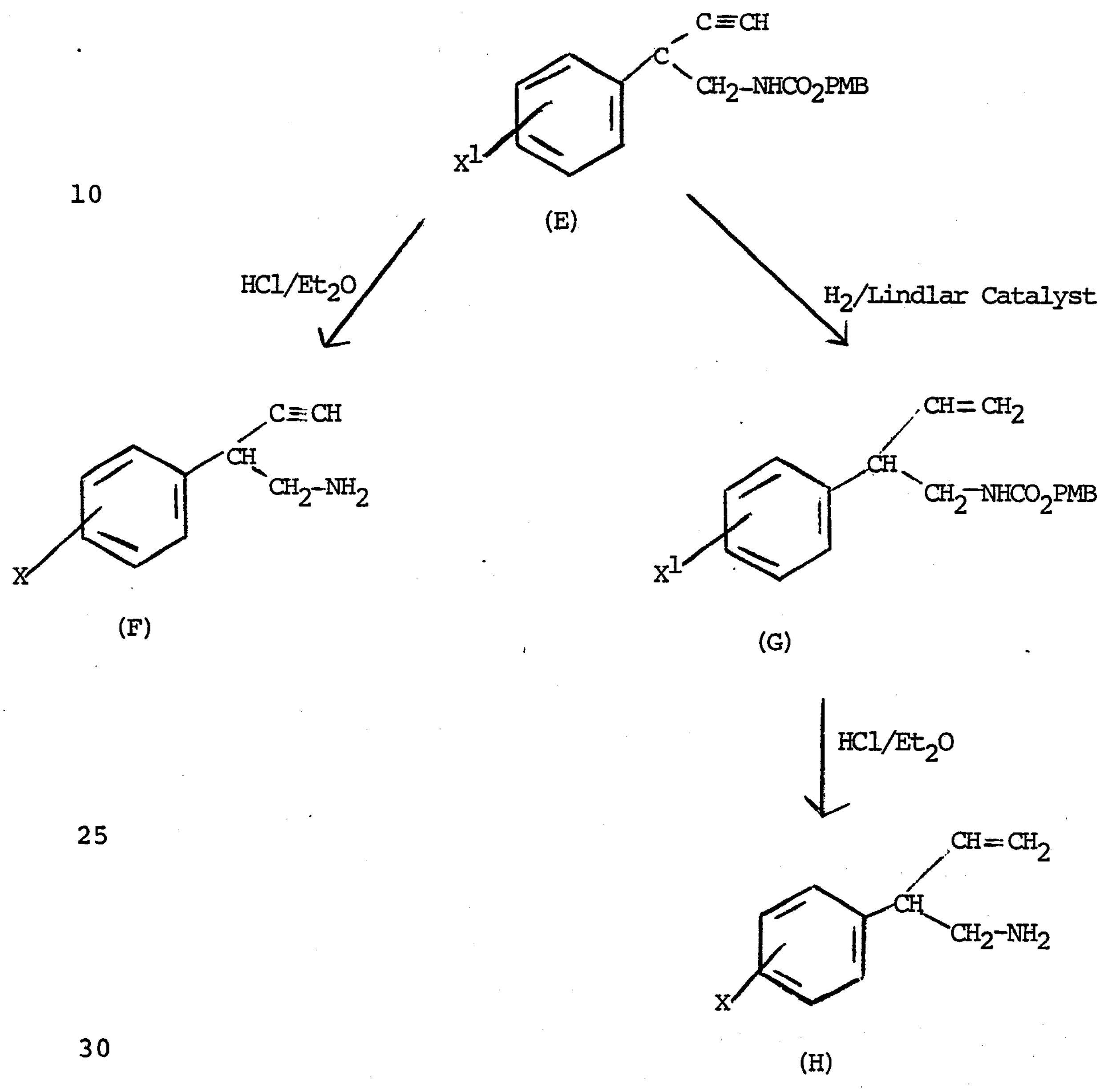


(E)

(F)

(G)

(H)

The pharmaceutically acceptable acid addition salts of the Formula (I) compounds are formed with strong or moderately strong organic or inorganic acids by methods known in the art. For example, the base is reacted with an inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts.

Presently invented also are methods of producing DBH inhibition in mammals, including humans, by administering an effective amount of a ß-ethynyl or ß-ethenyl benzeneethamine compound of Formula (I).

_In vitro_ enzyme inhibition kinetic studies demonstrate that the compounds useful in the methods of the invention are potent DBH inhibitors. DBH activity was assayed by a standard procedure for measuring the conversion of tyramine to octopamine in the presence of DBH. J.J. Pisano, _et al._, _Biochim. Biophy. Acta_, _43_, 566-68 (1960). Octopamine was assayed following sodium periodate oxidation to p-hydroxybenzaldehyde by measuring spectrophotometric absorbance at 330 nm.

To measure DBH inhibition, the enzyme was incubated for up to 50 minutes with 10, 20, 40, or 80 M concentrations of ß-ethynyl-4-hydroxybenzeneethanamine. This compound of the invention produced concentration-dependent DBH inhibition. Additionally, the enzyme inhibition was time-dependent which indicates that compounds of the invention are irreversible DBH inhibitors.

In vivo studies demonstrate that compounds useful in the methods of the invention effectively reduce blood pressure. Spontaneously hypertensive rats were dosed with a suspension or solution of ß-ethynylbenzeneethanamine methane sulfonate at a dose of 100 mg/kg intraperitoneally, and mean arterial blood pressure was monitored for 260 minutes using indwelling cannulae positioned in the tail arteries. Approximate twenty percent reductions in blood pressure were observed twenty to forty minutes following administration of this compound. At 260 minutes after administration of this compound, blood pressure remained reduced by approximately twenty percent when compared to vehicle-treated controls.

Blood pressure also was monitored in spontaneously hypertensive rats given 100 mg/kg intraperitoneally ß-ethynyl-3-hydroxybenzeneethanamine hydrochloride. Forty minutes following compound administration approximate thirty percent blood pressure reductions were observed. At 260 minutes after compound administration blood pressure remained decreased approximately ten percent.

The compounds useful in the methods of this invention can be incorporated into convenient dosage forms such as capsules, tablets or injectable preparations. Solid or liquid pharmaceutical carriers can be employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, emulsion, soft gelatin capsule,

sterile injectable liquid such as an ampoule, or an aqueous or nonaqueous liquid suspension.

The pharmaceutical preparations are made following conventional techniques of a pharmaceutical chemist involving mixing, granulating and compressing, when necessary, for tablet forms, or mixing, filling and dissolving the ingredients, as appropriate, to give the desired oral or parenteral products.

Doses of the present compounds in a pharmaceutical dosage unit will be an efficacious, nontoxic quantity selected from the range of 0.1-1,000 mg/kg of active compound, preferably 10-100 mg/kg. The selected dose is administered orally, rectally, or by injection from one to six times daily, or continuously by infusion to a human patient in need of treatment. Parenteral administration, which uses lower dosages, is preferred. Oral administration, at higher dosages, however, also can be used when safe and convenient for the patient.

The following examples are illustrative of preparation of ß-ethynyl and ß-ethenyl benzeneethanamine compounds and pharmaceutical compositions including these compounds. The examples are not intended to limit the scope of the invention as defined hereinabove and as claimed below. All temperatures and melting points (mp) are given in degrees Celsius (°C).

## EXAMPLE 1

### ß-Ethynylbenzeneethanamine

A mixture of Mg turnings (1.4 g, 0.058 g-atom) and THF (75 ml) was stirred under argon while a solution bromoethane (7.3 g, 0.067 mol) in THF (10 ml) was added. Iodomethane (0.05 ml) was added to initiate the reaction and after the dissolution of the Mg turnings ceased (30-40 min), a solution of trimethylsilylacetylene (6.34 g, 0.065

mol) in THF (20 ml) was added, and the resulting solution was stirred at ambient temperature for one hour. A solution of phenylmethylenepropanedioic acid diethyl ester (0.029 mol) in THF (100 ml) was added and the resulting solution was stirred until the phenylmethylenepropanedioic acid diethyl ester had completely reacted as judged by TLC. The reaction mixture was poured into saturated aqueous ammonium chloride and the product was extracted with ethyl acetate. The organic extracts were washed with water, and dried to afford a 60% yield of phenyl-(trimethylsilylethynyl)methylpropanedioic acid diethyl ester.

A solution of phenyl(trimethylsilylethynyl)methyl propanedioic acid diethyl ester (0.014 mol) in ethanol (45 ml) was stirred during the addition of a solution of sodium hydroxide (1.68 g, 0.042 mol) in water (105 ml) and then heated at 100°C until a clear solution resulted. The aqueous layer was cooled and acidified to pH 2 with 12N HCl, and the product was extracted with ethyl acetate. The ethyl acetate extracts were washed with water, dried, and concentrated to yield ß-ethynylbenzenepropanoic acid. The crude acid was heated at 170°C under argon until the evolution of carbon dioxide ceased. The cooled melt was dissolved in ethyl acetate (50 ml) and the resulting solution was extracted with 10% aqueous sodium hydroxide solution. The ethyl acetate extracts were washed with water, dried, treated with activated carbon to yield (76%) ß-ethynylbenzenepropanoic acid as a crystalline solid: mp 83-86°C.

(2-Ethynyl-2-phenylethyl)carbamic acid, 4-methoxyphenylmethyl ester was prepared in 52% yield by heating at 100°C for eight hours ß-ethynylbenzene-propanoic acid with diphenylphosphoryl azide (1 eq.), triethylamine (1 eq.), and 4-methoxybenzyl alcohol (1 eq.). The reaction mixture was cooled, concentrated, and

partitioned between water and ethyl acetate. The ethyl acetate was concentrated to give a solid residue: mp. 88-89°C.

(2-Ethynyl-2-phenylethyl)carbamic acid, 4-methoxyphenylmethyl ester then was deprotected by the following procedure. A solution of the carbamate (1 gm) in 1:1 ethyl acetate:ether (60 ml) was stirred during the addition of a saturated ethereal HCl solution (20 ml) and stirring was continued until the solution became cloudy. The solution was allowed to stand at ambient temperature until crystallization was complete. Recrystallization from methanol:ethyl acetate yielded (63%) ß-ethynylbenzeneethanamine hydrochloride: mp. 206-208°C.

EXAMPLE 2

ß-Ethynyl-3-Hydroxybenzeneethanamine

(3-Hydroxyphenylmethylene)propanedioic acid diethyl ester was alkylated with 4-methoxybenzyl chloride by treatment with sodium hydride in DMF. The crude product was purified by flash chromatography using 3:1 hexane:ethyl acetate as eluant to yield (85%) [3-(4-methoxyphenylmethoxy)phenylmethylene]propanedioic acid, diethyl ester.

The reaction of [3-(4-methoxyphenylmethoxy)phenylmethylene] propanedioic acid diethyl ester with trimethylsilylacetylene magnesium bromide prepared as in Example 1 yielded 47% of [3-(4-methoxyphenylmethoxy)phenyl]-3-(trimethylsilylethynyl)methylpropanedioic acid diethyl ester as an oil after substantial purification by flash chromatography using 5:1 hexane:ethyl acetate.

A solution of the propanedioic acid diethyl ester (6.77 g, 0.014 mol), prepared as above, in ethanol (45 ml) was stirred during the addition of a solution of sodium hydroxide (1.68 g, 0.042 mol) in water (105 ml) and then heated at 100°C until a clear solution resulted. The

solution was stirred an additional 1 hr at ambient temperature and then diluted with water and extracted with ethyl acetate. The aqueous layer was cooled and acidified to pH 2 with 12N HCl, and the product was extracted with ethyl acetate. The ethyl acetate extracts were washed with water, dried, and concentrated to yield 3.95 g (66%) of [3-(4-methoxyphenylmethoxy)phenyl](trimethylsilyl-ethynyl)methylpropanedioic acid. A solution of crude diacid (5.49 g, 0.013 mol) in 3:1 pyridine:water (50 ml) was heated to 100°C for 1 hr. The reaction mixture was cooled, carefully acidified to pH 2 with 12N HCl, and extracted with ethyl acetate. The organic extracts were dried and concentrated and the residue was dissolved in a minimum amount of ethanol containing sodium hydroxide (0.52 g, 0.013 mol). The ethanol solution was diluted with water (100 ml) and extracted with ethyl acetate. The aqueous phase was cooled and acidified to pH 2 with 12N HCl and the product was extracted with ethyl acetate. The ethyl acetate extracts were washed with water, dried, treated with activated carbon, and concentrated to yield 3.32 g (65%) of ß-(ethynyl-3-(4-methoxyphenylmethoxy)-benzenepropanoic acid: mp. 141-142°C.

The reaction of the benzenepropanoic acid prepared above in a procedure analogous to that used in Example 1 yielded [2-ethynyl-2-[3-(4-methoxyphenylmethoxy)-phenyl]ethyl]carbamic acid 4-methoxyphenylmethyl ester (35%): mp. 64-67°C.

A solution of [ß-ethynyl-2-[3-(4-methoxyphenyl-methoxy)phenyl]ethyl]carbamic acid, 4-methoxyphenylmethyl ester (0.90 g, 1.9 mmol) in ethyl acetate (20 ml) was treated with saturated ethereal HCl solution (2 ml) and stirred at ambient temperature until the reaction was complete as judged by TLC. Ether was added, the precipitate was filtered and dissolved in ethanol (3 ml) and this solution was added to a suspension of silver

acetate (0.315 g., 1.9 mmol) in ethanol (4 ml) and the mixture was stirred for 3 minutes and filtered. The filtrate was mixed with a solution of oxalic acid (0.171 g, 1.9 mmol) and the resulting solution was concentrated under reduced pressure. The residue was triturated with acetonitrile and recrystallized from methanol--acetonitrile to yield 0.117 g (25%) of ß-ethynyl-3-hydroxybenzeneethanamine, oxalate: mp. 176-178°C

A solution of the carbamate prepared above was deprotected using ethereal hydrochloride as in Example 1 to yield ß-ethynylbenzeneethanamine hydrochloride (63%): mp. 206-208°C.

EXAMPLE 3

ß-Ethynyl-4-Hydroxybenzeneethanamine Hydrochloride

A solution of 2,2-dimethyl-1,3-dioxane-4,6-dione (14.4 g, 0.10 mol) and 4-(4-methoxybenzyloxy)benzaldehyde (24.2 g, 0.10 mol) in toluene (100 ml) was treated with glacial acetic acid (2 ml) and piperidine (1 ml). The solution was stirred and heated at reflux until Dean-Stark removal of water was complete. The reaction mixture was cooled and the resulting solid orange product was filtered and washed with cold toluene to yield 24.9 g (68%) of 2,2-dimethyl-5-[4-(4-methoxyphenylmethoxy)phenylmethylene]-1,3-dioxane-4,6-dione: mp. 155-157°C.

A solution of the 1,3-dioxane-4,6-dione (10.7 g, 0.029 mol), prepared above, in THF (100 ml) was added to a solution of trimethylsilylacetylene magnesium bromide prepared as in Example 1, and the resulting solution was stirred until the reaction was complete as judged by TLC. The reaction mixture was poured into saturated aqueous ammonium chloride and the product was extracted with ethyl acetate. The organic extracts were washed with water, dried, and concentrated to yield 13.2 g (100%) of 2,2-dimethyl-5[4-(4-methoxyphenylmethoxy)phenyl)

(trimethylsilylethynylmethyl]-1,3-dioxane-4,6-dione: mp. 132-135°C.

A solution of the trimethylsilylethynylmethyl-1,3-dioxane-4,6-dione (14.2 g, 0.131 mol), prepared as above, in 3:1 pyridine:water (120 ml) was heated at 100°C for 4 hr then cooled in ice. The cold solution was carefully acidified to pH 2 with 12N HCl and extracted with ethyl acetate. The organic extracts were washed with water, dried, treated with activated carbon and concentrated to yield 8.43 g (63%) of [4-(4-methoxyphenylmethoxy)-phenyl](trimethylsilylethynyl)methylpropanoic acid intermediate: mp. 98-101°C. An analytical sample of the dicyclohexylammonium salt of this acid was prepared and recrystallized from acetonitrile: mp. 148-149°C.

A solution of crude trimethylsilylalkyne (8.43 g, 0.02 mol) and potassium fluoride (1.65 g, 0.029 mol) was heated at 50°C for 90 minutes in DMF (50 ml). The reaction mixture was cooled, filtered, and the solid potassium salt was washed with diethyl ether. A suspension of the potassium salt in water (50 ml) was stirred and acidified to pH 2 with 12N HCl, and the product was extracted with ethyl acetate. The organic extracts were washed with water, dried, and concentrated to yield 5.44 g (70%) of ß-ethynyl-4-(4-methoxyphenyl-methoxy)benzenepropanoic acid: mp. 154-156°C. This acid was characterized further as the dicyclohexylamine which crystallized from methanol:acetonitrile: mp. 153-156°C.

The reaction of the acid prepared above in a procedure analogous to that used in Example 1 yielded [2-ethynyl-2-[4-(4-methoxyphenylmethoxy)phenyl]ethyl]carbamic acid, 4-methoxyphenylmethyl ester. An analytical sample was prepared by recrystallization from ethylacetate: hexane: mp. 112-114°C.

Deprotection of the above carbamate by the procedure of Example 1 yielded ß-ethynyl-4-hydroxybenzene-

ethanamine hydrochloride (53%) which was recrystallized from methanol:ethyl acetate: mp. 160-163°C. An analytical sample of this compound was prepared by flash chromatography using 75:2 ethyl acetate:14N ammonium hydroxide as eluant followed by reconversion to the hydrochloride and recrystallization from methanol: acetonitrile: mp. 171-173°C.

EXAMPLE 4

ß-Ethenylbenzeneethanamine Hydrochloride

A solution (2-ethynyl-2-phenylethyl)carbamic acid, 4-methoxyphenylmethyl ester (1.55 g, 5 mmol), prepared as in Example 1, in dichloromethane (50 ml) was hydrogenated at ambient pressure over Aldrich Lindlar catalyst (palladium on calcium carbonate poisoned with lead) (230 mg) until conversion to product was complete (typically 90 minutes) as evidenced by TLC. The mixture was filtered, the filtrate was concentrated, and the residue was purified by flash chromatography using 3:1 hexane:ethyl acetate as eluant to yield 1.46 g (94%) of (2-ethenyl-2-phenylethyl)carbamic acid, 4-methoxyphenylmethyl ester. An analytical sample of this compound was prepared by repeated flash chromatography using 4:1 hexane:ethyl acetate as eluant: mp. 53-55°C.

Deprotection of the above carbamate by the procedure described in Example 1 yielded ß-ethynylbenzeneethanamine hydrochloride (38%), after recrystallization from ethyl acetate: mp. 113-116°C.

EXAMPLE 5

Hydrogenation of [2-ethynyl-2-[3-(4-methoxyphenylmethoxy)phenyl]ethyl]carbamic acid, 4-methoxyphenylmethyl ester as in Example 4 yielded [2-ethenyl-2-[3-(4-methoxyphenylmethoxy)phenyl]ethyl]carbamic acid, 4-methoxyphenylmethyl ester.

Deprotection of the above carbamate by the procedure described in Example 1 yielded ß-ethenyl-3-hydroxybenzeneethanamine hydrochloride (29%), after recrystallization from methanol:ethyl acetate: mp. 153-155°C.

EXAMPLE 6

ß-Ethenyl-4-Hydroxybenzeneethanamine Hydrochloride

Hydrogenation of [2-ethynyl-2-[4-(4-methoxyphenylmethoxy)phenyl]ethyl] carbamic acid, 4-methoxyphenylmethyl ester as in Example 4 yielded [2-ethenyl-2-[4-(4-methoxyphenylmethoxy)phenyl]ethyl]carbamic acid, 4-methoxyphenylmethyl ester (65%) which was purified by flash chromatography with 3:1 hexane:ethyl acetate as eluant.

The above carbamate was deprotected by the procedure of Example 1 to yield ß-ethenyl-4-hydroxybenzeneethanamine hydrochloride (38%), after recrystallization from methanol:ethyl acetate: mp. 168-170°C.

EXAMPLE 7

An oral dosage form for administering the presently invented compounds is produced by screening, mixing, and filling into a hard gelatin capsule the ingredients in the proportions shown in Table III, below.

Table III

| Ingredients | Amounts |
|---|---|
| ß-ethynylbenzeneethanamine hydrochloride | 50 mg |
| magnesium stearate | 5 mg |
| lactose | 75 mg |

EXAMPLE 8

The sucrose, calcium sulfate dihydrate and ß-ethynylbenzeneethanamine shown in Table IV below, are mixed and granulated in the proportions shown with a 10%

gelatin solution. The wet granules are screened, dried, mixed with the starch, talc and stearic acid, screened and compressed into 2 tablets.

Table IV

| Ingredients | Amounts |
|---|---|
| ß-ethynyl-3-hydroxybenzeneethanamine oxalate | 100 mg |
| calcium sulfate dihydrate | 150 mg |
| sucrose | 20 mg |
| starch | 10 mg |
| talc | 5 mg |
| stearic acid | 3 mg |

EXAMPLE 9

ß-ethynyl-3-hydroxybenzeneethanamine hydrochloride, 75 mg, is dispursed in 25 ml of normal saline to prepare an injectable preparation.

While the preferred embodiments of the invention are illustrated by the above, it is to be understood that the invention is not limited to the precise instructions herein disclosed and that the right to all modifications coming within the scope of the following claims is reserved.

Claims for Contracting States: BE, CH, DE, FR, GB, IT, LI, LU, NL, and SE

1. A compound of the Formula:

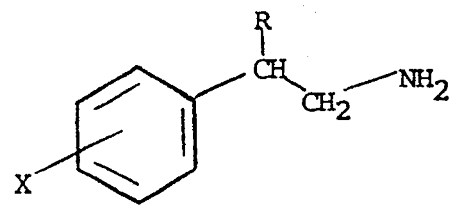

in which:

X is hydrogen or hydroxy; and

R is ethynyl or ethenyl; or

a pharmaceutically acceptable salt thereof.

2. A compound of Claim 1 that is ß-ethynylbenzeneethanamine or its hydrochloric acid salt, ß-ethynyl-3-hydroxybenzeneethanamine or its oxalic acid salt, ß-ethynyl-4-hydroxybenzeneethanamine or its hydrochloric acid salt, ß-ethenylbenzeneethanamine or its hydrochloric acid salt, ß-ethenyl-3-hydroxybenzeneethanamine or its hydrochloric acid salt, or ß-ethynyl-4-hydroxybenzeneethanamine or its hydrochloric acid salt.

3. A pharmaceutical composition comprising a pharmaceutically acceptable carrier and a compound of Claim 1.

4. A pharmaceutical composition of Claim 3 wherein the compound is ß-ethynylbenzeneethanamine or its hydrochloric acid salt, ß-ethynyl-3-hydroxybenzeneethanamine or its oxalic acid salt, ß-ethynyl-4-hydroxybenzeneethanamine or its hydrochloric acid salt, ß-ethenylbenzeneethanamine or its hydrochloric acid salt, ß-ethenyl-3-hydroxybenzeneethanamine or its hydrochloric acid salt, or ß-ethenyl-4-hydroxybenzeneethanamine or its hydrochloric acid salt.

5. A compound of the Formula:

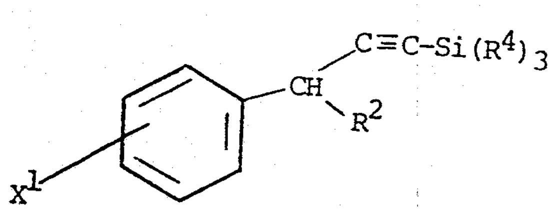

in which:

$X^1$ is H or 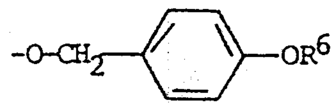

$R^6$ is $C_{1-4}$ alkyl;

$R^2$ is $CH(CO_2R^7)_2$; or 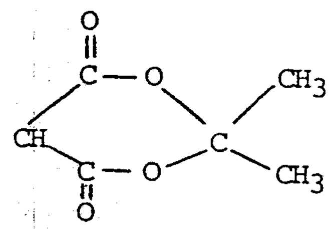 ;

$R^7$ is $C_{1-4}$ alkyl; and

$R^4$ is $C_{1-4}$ alkyl

provided that when $X^1$ is 4- 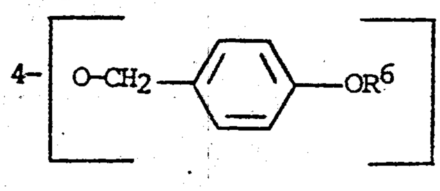

$R^2$ is 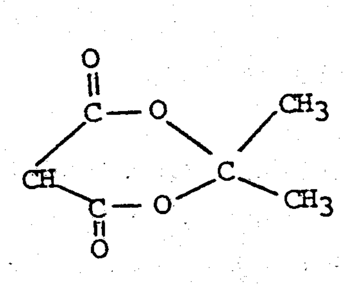

6. A compound of Claim 5 that is phenyl (trimethylsilylethynyl)methylpropanedioic acid diethyl ester, [3-(4-methoxyphenylmethoxy)]-3-(trimethylsilylethynyl)methylpropanedioic acid diethyl ester, or 2,2-dimethyl-5-[4-(4-methoxyphenylmethoxy)phenyl]-(trimethylsilylethynylmethyl)-1,3-dioxane-4,6-dione.

7. A compound of the Formula:

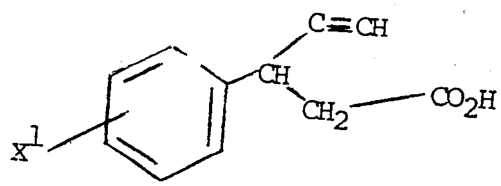

in which:

$X^1$ is H or 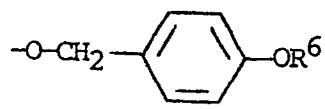 , and

$R^6$ is $C_{1-4}$ alkyl.

8. A compound of Claim 7 that is ß-ethynylbenzenepropanoic acid, ß-ethynyl-3-(4-methoxyphenylmethoxy)benzenepropanoic acid, or ß-ethynyl-4-(4-methoxyphenylmethoxy)benzenepropanoic acid.

9. A compound of the Formula:

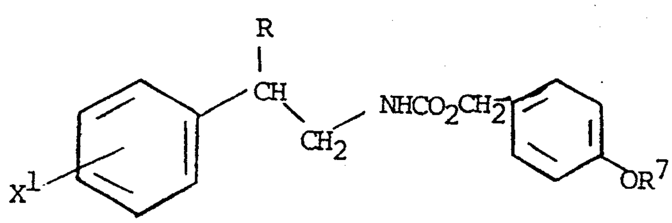

in which:

$X^1$ is H or 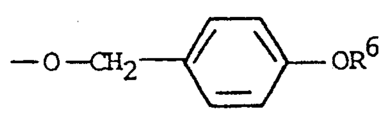 ;

$R^6$ and $R^7$ are $C_{1-4}$ alkyl; and

R is $-C\ \ CH$ or $-CH\equiv CH_2$.

10. A compound of Claim 9 that is 2-ethynyl-2-phenylethylcarbamic acid, 4-methoxyphenylmethyl ester, 2-[ethynyl-2-[3-(4-methoxyphenylmethoxy)phenyl]ethyl]-

carbamic acid, 4-methoxyphenylmethyl ester, 2-[ethynyl-2-[4-(4-methoxyphenylmethoxy)phenyl]ethyl]carbamic acid, 4-methoxyphenylmethyl ester, 2-ethenyl-2-phenylethyl-carbamic acid, 4-methoxyphenylmethyl ester, [2-ethenyl-2-[3-(4-methoxyphenylmethoxy)phenyl]ethyl]carbamic acid, 4-methoxyphenylmethyl ester, or 2-[ethenyl-2-(4-(4-methoxy phenylmethoxy)phenyl]ethyl]carbamic acid, 4-methoxyphenylmethyl ester.

11. A process for preparing a compound of the formula:

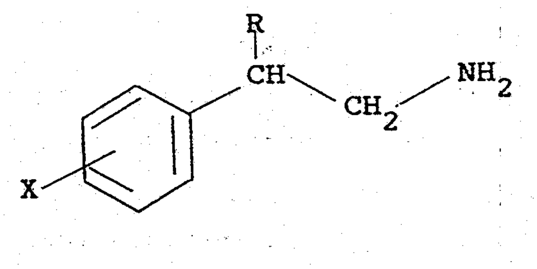

in which

X is hydrogen or hydroxy; and
R is ethenyl or ethynyl; or

a pharmaceutically acceptable salt thereof, that comprises deprotecting a compound of formula (XXX):

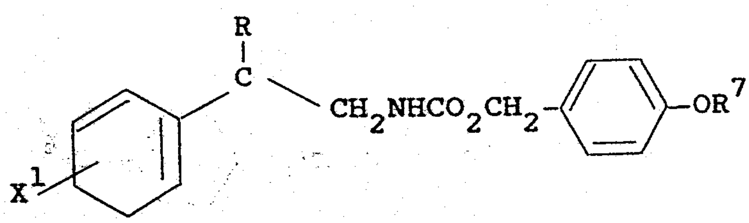 (XXX)

in which

R is ethenyl or ethynyl;

$X^1$ is H or 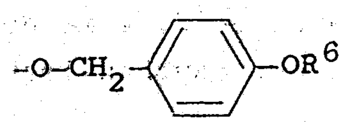, and

$R^6$ and $R^7$ are $C_{1-4}$alkyl, and optionally thereafter forming a pharmaceutically acceptable salt.

12. A process for preparing compounds of the formula (XX):

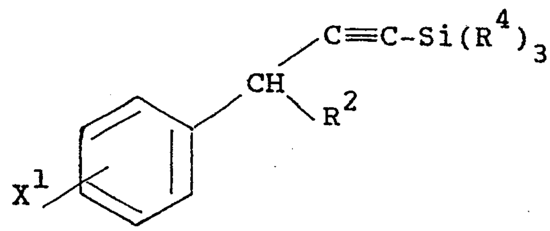

(XX)

in which

$X^1$ is -H or 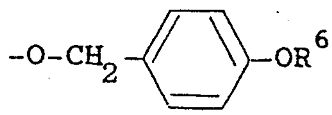

$R^6$ is $C_{1-4}$ alkyl;
$R^4$ is $C_{1-4}$ alkyl; and
$R^2$ is $-CH(CO_2R^5)_2$ or 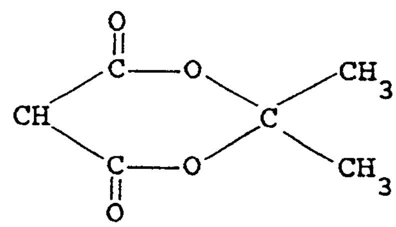

$R^5$ is $C_{1-4}$alkyl

provided that when $X^1$ is 4- 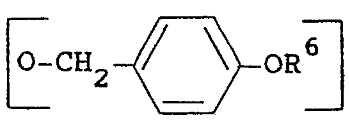

$R^2$ is 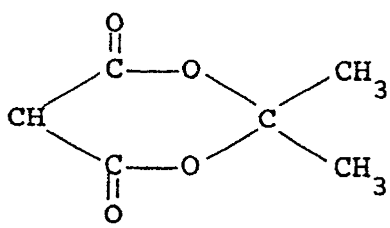

that comprises reaction of a compound of formula (XXI):

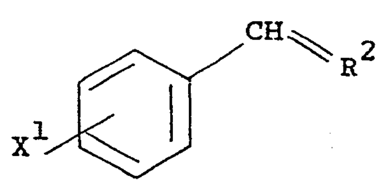

(XXI)

wherein $X^1$ and $R^5$ are as in formula (XX) and $R^2$ is $C(CO_2R^5)_2$ or

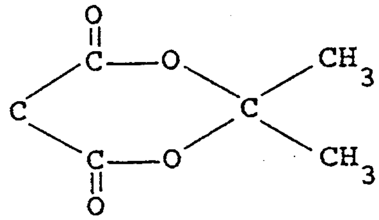

with a compound of formula (XXII)

$$(R^4)_3\text{-Si-C}\equiv\text{C-MgZ} \qquad \text{(XXII)}$$

wherein $R^4$ is as in formula (XX), and Z is Br, Cl, F, or I; followed by addition of the reaction mixture to aqueous acid.

13. A process for preparing compounds of the formula (X):

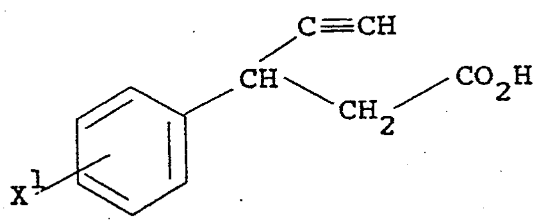

(X)

in which

$X^1$ is -H or -O-$CH_2$- 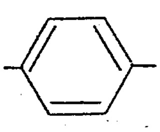 -$OR^6$ and

$R^6$ is $C_{1-4}$ alkyl, that comprises reaction of a compound of the formula:

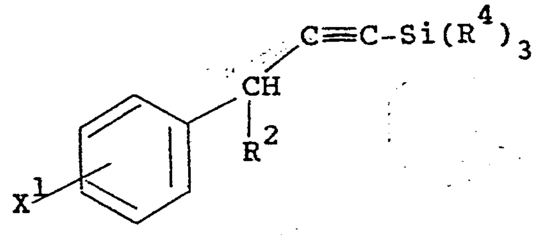

in which $X^1$ and $R^2$ are as described in claim 12, $R^4$ is $C_{1-4}$alkyl with a suitable base followed by (a) where $R^2$ is $CH(CO_2R^5)_2$ acidification with a strong acid and heating the reaction mixture at about 80-120°C in the presence of an aqueous organic base, or (b) where $R^2$ is

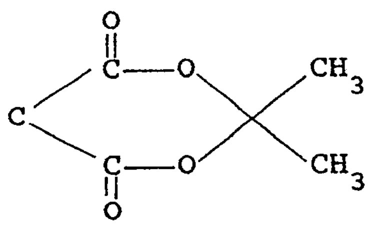

heating at about 40-60°C om dipolar, aprotic solvent an organic extract of the reaction mixture with potassium fluoride followed by acidification by addition of a strong acid.

14. A compound of claim 1 for use as a therapeutic agent.

15. A compound of claim 1 for use in the treatment of hypertension.

Claims for Contracting States : AT, GR and ES

1. A process for preparing a compound of the formula:

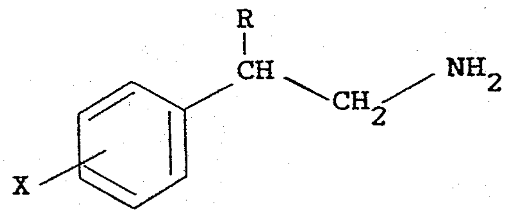

(I)

in which

X is hydrogen or hydroxy; and
R is ethenyl or ethynyl; or

a pharmaceutically acceptable salt thereof, that comprises deprotecting a compound of formula (XXX):

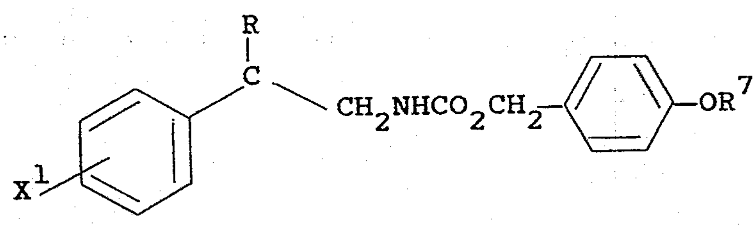

(XXX)

in which

R is ethenyl or ethynyl;

$X^1$ is H or 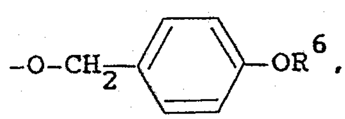, and

$R^6$ and $R^7$ are $C_{1-4}$alkyl, and optionally thereafter forming a pharmaceutically acceptable salt.

2. A process according to claim 1 wherein the compound prepared is
β-ethynylbenzeneethanamine or its hydrochloric acid salt;
β-ethynyl-3-hydroxybenzeneethanamine or its oxalic acid salt;
β-ethynyl-4-hydroxybenzeneethanamine or its hydrochloric acid salt;
β-ethenylbenzeneethanamine or its hydrochloric acid salt;
β-ethenyl-3-hydroxybenzeneethanamine or its hydrochloric acid salt, or
β-ethynyl-4-hydroxybenzeneethanamine or its hydrochloric acid salt.

3. A process for preparing compounds of the formula (XX):

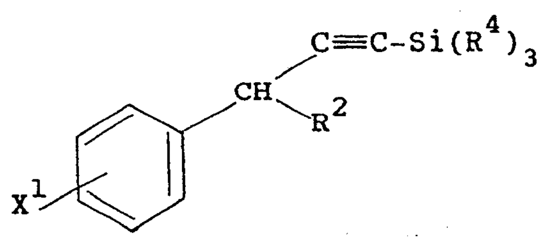

(XX).

in which

$X^1$ is -H or 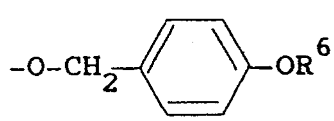

$R^6$ is $C_{1-4}$ alkyl;
$R^4$ is $C_{1-4}$ alkyl; and
$R^2$ is $-CH(CO_2R^5)_2$ or 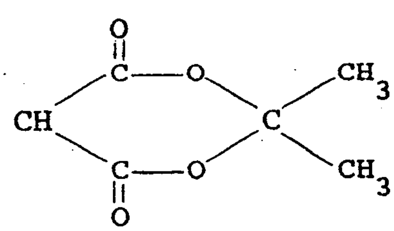

$R^5$ is $C_{1-4}$alkyl

provided that when $X^1$ is 4- 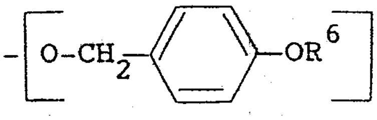

$R^2$ is 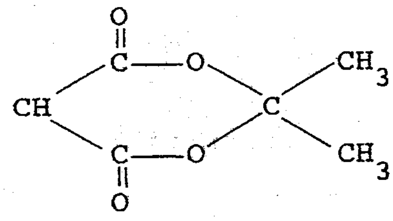

that comprises reaction of a compound of formula (XXI):

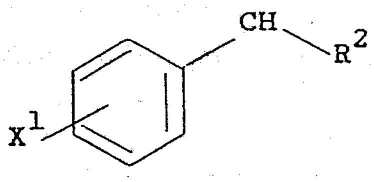

(XXI)

wherein $X^1$ and $R^5$ are as in formula (XX) and $R^2$ is $C(CO_2R^5)_2$ or 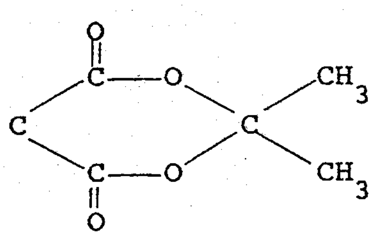

with a compound of formula (XXII)

$$(R^4)_3\text{-Si-C}\equiv\text{C-MgZ} \qquad \text{(XXII)}$$

wherein $R^4$ is as in formula (XX), and Z is Br, Cl, F, or I; followed by addition of the reaction mixture to aqueous acid.

4. A process for preparing compounds of the formula (X):

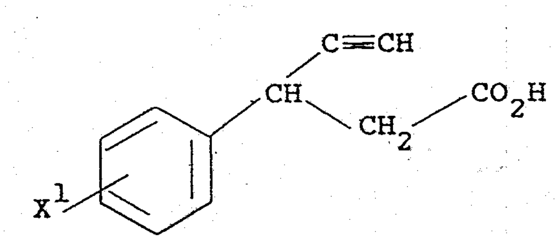

(X)

in which

$X^1$ is -H or $-O-CH_2-$ 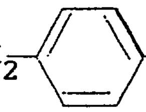 $-OR^6$ and

$R^6$ is $C_{1-4}$ alkyl, that comprises reaction of a compound of the formula:

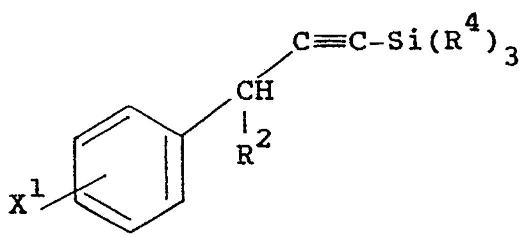

in which $X^1$ and $R^2$ are as described in claim 12, $R^4$ is $C_{1-4}$alkyl with a suitable base followed by (a) where $R^2$ is $CH(CO_2R^5)_2$ acidification with a strong acid and heating the reaction mixture at about 80-120°C in the presence of an aqueous organic base, or (b) where $R^2$ is

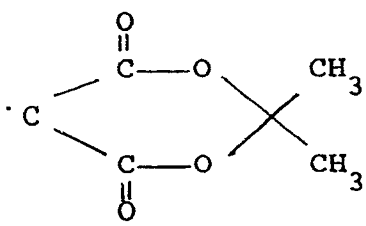

heating at about 40-60°C om dipolar, aprotic solvent an organic extract of the reaction mixture with potassium fluoride followed by acidification by addition of a strong acid.

5. A process for the preparation of a pharmaceutical composition which comprises contacting a compound of structure (I) as described in claim 1 and a pharmaceutically acceptable carrier.

6. A process according to claim 5 in which the compound of structure (I) is

ß-ethynylbenzeneethanamine or its hydrochloric acid salt;

ß-ethynyl-3-hydroxybenzeneethanamine or its oxalic acid salt;

ß-ethynyl-4-hydroxybenzeneethanamine or its hydrochloric acid salt;

ß-ethenylbenzeneethanamine or its hydrochloric acid salt,

ß-ethenyl-3-hydroxybenzeneethanamine or its hydrochloric acid salt, or

ß-ethynyl-4-hydroxybenzeneethanamine or its hydrochloric acid salt.

0250264

European Patent Office

# EUROPEAN SEARCH REPORT

Application number

EP 87305476.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A2 - 0 145 361 (MCNEILAB)<br>* Claims 1,10,11; page 6, line 13 - page 9, line 13 *<br>-- | 1,3,7, 9,14, 15 | C 07 C 87/29<br>C 07 C 93/14<br>C 07 C 85/20<br>C 07 C 57/42<br>C 07 C 59/66<br>C 07 C 51/00<br>C 07 F 7/08<br>C 07 C 125/065<br>A 61 K 31/135 |
| D,A | ANNALS OF THE NEW YORK ACADEMY OF SCIENCES, vol. 107, July 1963, New York<br>B.M.BLOOM "Some structural considerations regarding compounds that influence monoamine metabolism" pages 878-890<br>* Page 878, line 1 - page 879, line 20; page 885, line 30 - page 889, line 3 *<br>-- | 1,14 | |
| A | EP - A1 - 0 011 608 (ASTRA LÄKEMEDEL AKTIEBOLAG)<br>* Claims 1-3; abstract *<br>-- | 1,3,14 | **TECHNICAL FIELDS SEARCHED (Int. Cl.4)** |
| A | CHEMICAL ABSTRACTS, vol. 65, no. 2, July 18, 1966, Columbus, Ohio, USA<br>BURGER et al. "1-Ethynylphenetylamine"<br>column 3772, abstract-no. 3 772c<br>& J.Med.Chem. 9(4), 469-70(1966)<br>-- | 1,14 | C 07 C 87/00<br>C 07 C 93/00<br>C 07 C 85/00<br>C 07 C 57/00<br>C 07 C 59/00<br>C 07 F 7/00<br>C 07 C 125/00 |
| A | US - A - 4 537 974 (LAU)<br>* Formula sheet *<br>---- | 5,12, 13 | |

The present search report has been drawn up for all claims

| Place of search | Date of completion of the search | Examiner |
|---|---|---|
| VIENNA | 07-09-1987 | KÖRBER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82